# EUROPEAN PATENT SPECIFICATION

(11) **EP 1 701 958 B1**
(45) Date of publication and mention of the grant of the patent: **02.05.2007**
(21) Application number: 04798641.9
(22) Date of filing: 24.11.2004
(51) Int. Cl.: C07D 487/04, A61K 31/415

(54) **PYRROLO-PYRAZINE DERIVATIVES USEFUL AS CB1-MODULATORS**
ALS CB1-MODULATOREN GEEIGNETE PYRROLOPYRAZINDERIVATE
DERIVES DE PYRROLOPYRAZINES COMME MODULATEURS DE CB-1

(30) Priority: 25.11.2003 GB 0327331
(43) Date of publication of application: 20.09.2006
(73) Proprietor: AstraZeneca AB, 151 85 Södertälje (SE)
(72) Inventor: CHENG, Leifeng, S-431 83 Molndal (SE)
(86) International application number: PCT/GB2004/004934
(87) International publication number: WO 2005/051953

(56) References cited:
- EP-A- 0 656 354
- WO-A-01/70700
- SAITO, SHOICHI ET AL: "New Molecular Vessels: Synthesis and Chiroselective Recognition" JOURNAL OF THE AMERICAN CHEMICAL SOCIETY , 122(40), 9628-9630 CODEN: JACSAT; ISSN: 0002-7863, 2000, XP002325542

## Description

### Field of invention

The present invention relates to certain compounds of formula I, to processes for preparing such compounds useful in the treatment of obesity, psychiatric and neurological disorders and to pharmaceutical compositions containing them.

### Background of the invention

It is known that certain CB₁ modulators (known as antagonists or inverse agonists) are useful in the treatment of obesity, psychiatric and neurological disorders (WO01/70700 and EP 656354). However, there is a need for CB₁ modulators with improved physicochemical properties and/or DMPK properties and/or pharmacodynamic properties.

2,3-Diphenyl- 5H-pyrrolo[3,4-b]pyrazine-5,7(6H)-dione is disclosed in Agricultural and Biological Chemistry (1981), 45(9), 2129-30 and in Journal of Polymer Science, Polymer Chemistry Edition (1971), 9(4), 1117-38.

### Description of the invention

The invention relates to a compound of formula (I) 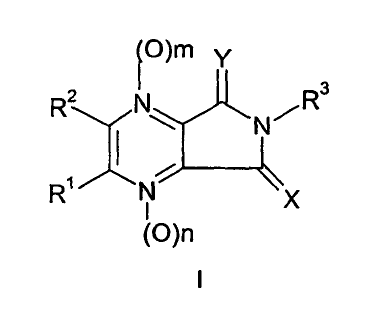 and pharmaceutically acceptable salts thereof, in which
R¹ and R² independently represent phenyl, thienyl, pyridyl, C₁₋₁₀alkyl, C₁₋₁₀alkoxy or C₃₋₁₅cycloalkyl;
R³ represents a C₁₋₁₅alkyl group, C₃₋₁₅cycloalkyl, a phenylC₁₋₄alkyl group, a heteroaryl group, a heteroarylC₁₋₄alkyl group, or a group R⁴(CH₂)ₙ- in which R⁴ represents a saturated or partially unsaturated 5 to 8 membered heterocyclic group containing one or more heteroatoms selected from nitrogen, oxygen or sulphur and n is 0, 1, 2, 3 or 4;
X and Y independently represent O or S;
m and n independently represent 0 or 1;
wherein each of R¹, R², R³ and R⁴ is optionally substituted by one, two or three groups represented by Z wherein Z represents a C₁₋₆alkyl group optionally substituted by one or more fluoro, a C₁₋₆alkoxy group optionally substituted by one or more fluoro, hydroxy, halo, trifluoromethylsulphonyl, benzyl, nitro, amino, mono or di C₁₋₄alkylamino, mono or di C₁₋₃alkylamido, C₁₋₃alkylsulphonyl, C₁₋₆alkoxycarbonyl, carboxy, cyano, carbamoyl, mono or di C₁₋₃alkyl carbamoyl, sulphamoyl or acetyl.

In a particular group of compounds of formula I, R¹ and R² independently represent phenyl, thienyl, pyridyl, C₁₋₁₀alkyl, C₁₋₁₀alkoxy or C₃₋₁₅cycloalkyl ;
R³ represents a C₁₋₁₅alkyl group, C₃₋₁₅cycloalkyl, a phenylC₁₋₄alkyl group, a heteroarylC₁₋₄alkyl group, or a group R⁴(CH₂)ₙ- in which R⁴ represents a saturated or partially unsaturated 5 to 8 membered heterocyclic group containing one or more heteroatoms selected from nitrogen, oxygen or sulphur and n is 0, 1, 2, 3 or 4;
X and Y independently represent O or S;
m and n independently represent 0 or 1;
wherein each of R¹, R², R³ and R⁴ is optionally substituted by one, two or three groups represented by Z wherein Z represents a C₁₋₆alkyl group optionally substituted by one or more fluoro, a C₁₋₆alkoxy group optionally substituted by one or more fluoro, hydroxy, halo, trifluoromethylsulphonyl, benzyl, nitro, amino, mono or di C₁₋₄alkylamino, mono or di C₁₋₃alkylamido, C₁₋₃alkylsulphonyl, C₁₋₆alkoxycarbonyl, carboxy, cyano, carbamoyl, mono or di C₁₋₃alkyl carbamoyl, sulphamoyl or acetyl.

A particular group of compounds of formula I is represented by formula IA 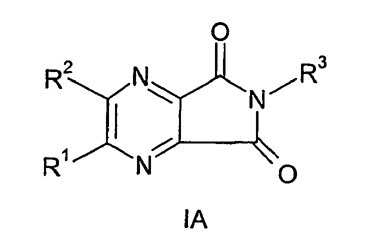 in which R¹, R² and R³ are as previously defined in either the first or second paragraph immediately above.

It will be understood that where a substituent Z is present in more than one group that these substituents are independently selected and may be the same or different.

The term C₃₋₁₅cycloalkyl includes monocyclic, bicyclic, tricyclic and spiro systems for example, cyclopentyl, cyclohexyl and adamantyl.

The term heteroaryl means an aromatic 5-, 6-, or 7-membered monocyclic ring or a 9- or 10-membered bicyclic ring, with up to five ring heteroatoms selected from oxygen, nitrogen and sulfur. Suitable aromatic heteroaryl groups include, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl. Preferably furyl, pyrrolyl, thienyl, oxazolyl, isoxazolyl, imidazolyl, pyrazolyl, oxazolyl thiazolyl, isothiazolyl, oxadiazolyl, thiadiazolyl, triazolyl, tetrazolyl, pyridyl, pyridazinyl, pyrimidinyl, pyrazinyl or 1,3,5-triazenyl and more preferably pyrrolyl, thienyl, imidazolyl, oxazolyl or pyridyl.

Suitable saturated or partially unsaturated 5 to 8 membered heterocyclic groups containing one or more heteroatoms selected from nitrogen, oxygen or sulphur include, for example oxiranyl, oxetanyl, tetrahydrofuranyl, tetrahydropyranyl, 2,3-dihydro-1,3-thiazolyl, 1,3-thiazolidinyl, pyrrolinyl, pyrrolidinyl, morpholinyl, tetrahydro-1,4-thiazinyl, 1-oxotetrahydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl or tetrahydropyrimidinyl, preferably tetrahydrofuranyl, tetrahydropyranyl, pyrrolidinyl, morpholinyl, piperidinyl or piperazinyl, more preferably tetrahydrofuran-3-yl, tetrahydropyran-4-yl, pyrrolidin-3-yl, morpholino, piperidino, piperidin-4-yl or piperazin-1-yl.

Particularly Z represents halo, trifluoromethyl, trifluoromethylthio, difluoromethoxy or trifluoromethoxy.

Further values of R¹, R², R³, and R⁴ in compounds of formula I and compounds of formula IA now follow. It will be understood that such values may be used where appropriate with any of the definitions, claims or embodiments defined hereinbefore or hereinafter.

R¹ and R² independently represent phenyl optionally substituted independently by one or two halo. Particularly R¹ and R² are identical. More particularly R¹ and R² each represent 4-chlorophenyl.

R³ represents a phenylC₁₋₄alkyl, pyridylC₁₋₄alkyl, a C₁₋₆alkyl group, piperidino, morpholino, pyrrolidino wherein the phenyl ring is optionally substituted by halo and the pyridyl ring is optionally substituted by one or more of the following :halo, a C₁₋₆alkyl group (optionally substituted by one or more fluoro for example trifluoromethyl), or a C₁₋₆alkoxy group (optionally substituted by one or more fluoro for example difluoromethoxy or trifluoromethoxy). Particularly R³ represents benzyl, halobenzyl, pyridylmethyl, piperidino or a C₃₋₄alkyl group. More particularly R³ represents 4-fluorobenzyl, 4-pyridylmethyl, piperidino or *tert*-butyl.

X and Y both present O.

m and n are both 0.

In one group of compounds of formula I R¹ and R² independently represent phenyl optionally substituted independently by one or two chloro, R³ represents benzyl, halobenzyl, pyridylmethyl, piperidino or a C₃₋₄alkyl group, X and Y are both O and m and n are both 0.

"Pharmaceutically acceptable salt", where such salts are possible, includes both pharmaceutically acceptable acid and base addition salts. A suitable pharmaceutically acceptable salt of a compound of Formula I is, for example, an acid-addition salt of a compound of Formula I which is sufficiently basic, for example an acid-addition salt with an inorganic or organic acid such as hydrochloric, hydrobromic, sulphuric, trifluoroacetic, citric or maleic acid; or, for example a salt of a compound of Formula I which is sufficiently acidic, for example an alkali or alkaline earth metal salt such as a sodium, calcium or magnesium salt, or an ammonium salt,or a salt with an organic base such as methylamine, dimethylamine, trimethylamine, piperidine, morpholine or tris-(2-hydroxyethyl)amine.

Throughout the specification and the appended claims, a given chemical formula or name shall encompass all stereo and optical isomers and racemates thereof as well as mixtures in different proportions of the separate enantiomers, where such isomers and enantiomers exist, as well as pharmaceutically acceptable salts thereof and solvates thereof such as for instance hydrates. Isomers may be separated using conventional techniques, e.g. chromatography or fractional crystallisation. The enantiomers may be isolated by separation of racemate for example by fractional crystallisation, resolution or HPLC. The diastereomers may be isolated by separation of isomer mixtures for instance by fractional crystallisation, HPLC or flash chromatography. Alternatively the stereoisomers may be made by chiral synthesis from chiral starting materials under conditions which will not cause racemisation or epimerisation, or by derivatisation, with a chiral reagent. All stereoisomers are included within the scope of the invention. All tautomers, where possible, are included within the scope of the invention.

The following definitions shall apply throughout the specification and the appended claims.
Unless otherwise stated or indicated, the term "alkyl" denotes either a straight or branched alkyl group. Examples of said alkyl include methyl, ethyl, n-propyl, isopropyl, n-butyl, iso-butyl, sec-butyl and t-butyl. Preferred alkyl groups are methyl, ethyl, propyl, isopropyl and tertiary butyl.

Unless otherwise stated or indicated, the term "alkoxy" denotes a group O-alkyl, wherein alkyl is as defined above.

Unless otherwise stated or indicated, the term "halogen" shall mean fluorine, chlorine, bromine or iodine.

Specific compounds of the invention are one or more of the following: 2,3-bis(4-chlorophenyl)-6-(4-fluorobenzyl)-5H-pyrrolo[3,4-b]pyrazine-5,7(6H)-dione; 2,3-bis(4-chlorophenyl)-6-(pyridin-4-ylmethyl)-5*H*-pyrrolo[3,4-*b*]pyrazine-5,7(6*H*)-dione; 2,3-bis(4-chlorophenyl)-6-piperidin-1-yl-5*H*-pyrrolo[3,4-*b*]pyrazine-5,7(6*H*)-dione; or 6-*tert*-butyl-2,3-bis(4-chlorophenyl)-5*H*-pyrrolo[3,4-*b*]pyrazine-5,7(6*H*)-dione as well as pharmaceutically acceptable salts thereof

### Methods of preparation

The compounds of the invention may be prepared as outlined below according to any of the following methods. However, the invention is not limited to these methods, the compounds may also be prepared as described for structurally related compounds in the

### prior art.

Compounds of formula I in which m and n are each 0 and X and Y are both O are prepared by reacting a compound of formula II with a dehydrating agent for example oxalyl chloride optionally in the presence of a solvent for the compound of formula II at a temperature in the range of 0-100°C.

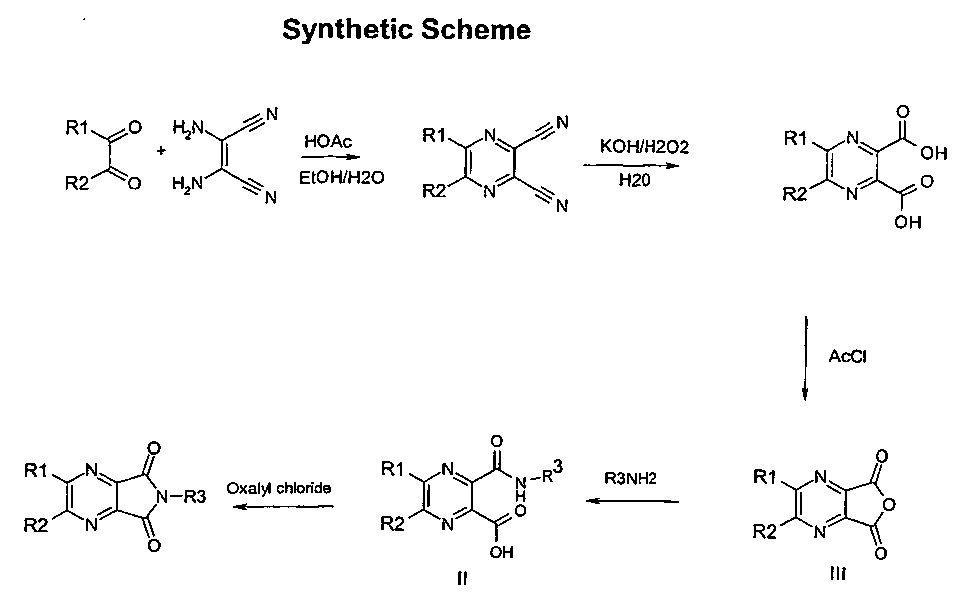

Compounds of formula I in which either X or Y or both X and Y are S may be prepared by reacting a compound of formula I in which X and Y are both O with an appropriate molar amount of Lawesson's reagent by methods known to those skilled in the art.
Compounds of formula I in which either of n and m is 1 or both are 1 may be prepared by oxidising a compound of formula I in which n and m are both 0 with an appropriate molar quantity of oxidising agent for example a peroxide e.g.hydrogen peroxide or sulphuric peroxide.

Certain compounds of formula II and III are believed to be novel and form part of the present invention.

### Pharmaceutical preparations

The compounds of the invention will normally be administered via the oral, parenteral, intravenous, intramuscular, subcutaneous or in other injectable ways, buccal, rectal, vaginal, transdermal and/or nasal route and/or via inhalation, in the form of pharmaceutical preparations comprising the active ingredient or a pharmaceutically acceptable addition salt, in a pharmaceutically acceptable dosage form. Depending upon the disorder and patient to be treated and the route of administration, the compositions may be administered at varying doses.

Suitable daily doses of the compounds of the invention in the therapeutic treatment of humans are about 0.001-10 mg/kg body weight, preferably 0.01-1 mg/kg body weight.

Oral formulations are preferred particularly tablets or capsules which may be formulated by methods known to those skilled in the art to provide doses of the active compound in the range of 0.5mg to 500mg for example 1 mg, 3 mg, 5 mg, 10 mg, 25mg, 50mg, 100mg and 250mg.

According to a further aspect of the invention there is also provided a pharmaceutical formulation including any of the compounds of the invention, or pharmaceutically acceptable derivatives thereof, in admixture with pharmaceutically acceptable adjuvants, diluents and/or carriers.

### Pharmacological properties

The compounds of formula (I) are useful for the treatment of obesity, psychiatric disorders such as psychotic disorders, schizophrenia, bipolar disorders, anxiety, anxio-depressive disorders, depression, cognitive disorders, memory disorders, obsessive-compulsive disorders, anorexia, bulimia, attention disorders like ADHD, epilepsy, and related conditions, and neurological disorders such as dementia, neurological disorders(e.g. Multiple Sclerosis), Raynaud's syndrome, Parkinson's disease, Huntington's chorea and Alzheimer's disease. The compounds are also potentially useful for the treatment of immune, cardiovascular, reproductive and endocrine disorders, septic shock and diseases related to the respiratory and gastrointestinal systems (e.g. diarrhea). The compounds are also potentially useful as agents in treatment of extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms. The compounds may also eliminate the increase in weight which normally accompanies the cessation of smoking.

In another aspect the present invention provides a compound of formula I as previously defined for use as a medicament.

In a further aspect the present invention provides the use of a compound of formula I in the preparation of a medicament for the treatment or prophylaxis of obesity, psychiatric disorders such as psychotic disorders, schizophrenia, bipolar disorders, anxiety, anxio-depressive disorders, depression, cognitive disorders, memory disorders, obsessive-compulsive disorders, anorexia, bulimia, attention disorders like ADHD, epilepsy, and related conditions, neurological disorders such as dementia, neurological disorders (e.g. Multiple Sclerosis), Parkinson's Disease, Huntington's Chorea and Alzheimer's Disease, immune, cardiovascular, reproductive and endocrine disorders, septic shock, diseases related to the respiratory and gastrointestinal systems (e.g. diarrhea), and extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms.

In a still further aspect the present invention provides a method of treating obesity, psychiatric disorders such as psychotic disorders such as schizophrenia and bipolar disorders, anxiety, anxio-depressive disorders, depression, cognitive disorders, memory disorders, obsessive-compulsive disorders, anorexia, bulimia, attention disorders like ADHD, epilepsy, and related conditions, neurological disorders such as dementia, neurological disorders (e.g. Multiple Sclerosis), Parkinson's Disease, Huntington's Chorea and Alzheimer's Disease, immune, cardiovascular, reproductive and endocrine disorders, septic shock, diseases related to the respiratory and gastrointestinal systems (e.g. diarrhea), and extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms comprising administering a pharmacologically effective amount of a compound of formula I to a patient in need thereof.

The compounds of the present invention are particulary suitable for the treatment of obesity, e.g. by reduction of appetite and body weight, maintenance of weight reduction and prevention of rebound.

The compounds of the present invention may also be used to prevent or reverse medication-induced weight gain, e.g. weight gain caused by antipsychotic (neuroleptic) treatment(s). The compounds of the present invention may also be used to prevent or reverse weight gain associated with smoking cessation.

The compounds of this invention may be useful in the inhibition of osteoclasts and the inhibition of bone resorption for the treatment of bone disorders, such as osteoporosis, for cancer associated bone disease and hypercalcemia of malignancies, and for treatment of Paget's bone disease. Compounds of this invention may also be useful in the treatment of other bone disorders with an inflammatory background such as osteo-arthritis, rheumatoid arthritis and for steroid/drug- induced osteoporosis.

### Combination Therapy

The compounds of the invention may be combined with another therapeutic agent that is useful in the treatment of disorders associated with the development and progress of obesity such as hypertension, hyperlipidaemias, dyslipidaemias, diabetes and atherosclerosis. For example, a compound of the present invention may be used in combination with a compound that affects thermogenesis, lipolysis, fat absorption, satiety, or gut motility. The compounds of the invention may be combined with another therapeutic agent that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

The compounds of the invention may be used alongside other therapies for the treatment of obesity and its associated complications the metabolic syndrome and type 2 diabetes, these include biguanide drugs, insulin (synthetic insulin analogues) and oral antihyperglycemics (these are divided into prandial glucose regulators and alpha-glucosidase inhibitors).

In another aspect of the invention, the compound of formula I, or a pharmaceutically acceptable salt thereof may be administered in association with a PPAR modulating agent. PPAR modulating agents include but are not limited to a PPAR alpha and/or gamma agonist, or pharmaceutically acceptable salts, solvates, solvates of such salts or prodrugs thereof. Suitable PPAR alpha and/or gamma agonists, pharmaceutically acceptable salts, solvates, solvates of such salts or prodrugs thereof are well known in the art.

In addition the combination of the invention may be used in conjunction with a sulfonylurea. The present invention also includes a compound of the present invention in combination with a cholesterol-lowering agent. The cholesterol-lowering agents referred to in this application include but are not limited to inhibitors of HMG-CoA reductase (3-hydroxy-3-methylglutaryl coenzyme A reductase). Suitably the HMG-CoA reductase inhibitor is a statin

In the present application, the term "cholesterol-lowering agent" also includes chemical modifications of the HMG-CoA reductase inhibitors, such as esters, prodrugs and metabolites, whether active or inactive.

The present invention also includes a compound of the present invention in combination with an inhibitor of the ileal bile acid transport system (IBAT inhibitor). The present invention also includes a compound of the present invention in combination with a bile acid binding resin.
The present invention also includes a compound of the present invention in combination with a bile acid sequestering agent, for example colestipol or cholestyramine or cholestagel According to an additional further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration one or more of the following agents selected from:
a CETP (cholesteryl ester transfer protein) inhibitor;
a cholesterol absorption antagonist;
a MTP (microsomal transfer protein) inhibitor;
a nicotinic acid derivative, including slow release and combination products;
a phytosterol compound;
probucol;
an anti-coagulant;
an omega-3 fatty acid;
another anti-obesity compound;
an antihypertensive compound for example an angiotensin converting enzyme (ACE) inhibitor, an angiotensin II receptor antagonist, an andrenergic blocker, an alpha andrenergic blocker, a beta andrenergic blocker, a mixed alpha/beta andrenergic blocker, an andrenergic stimulant, calcium channel blocker, an AT-1 blocker, a saluretic, a diuretic
or a vasodilator;
a Melanin concentrating hormone (MCH) antagonist;
a PDK inhibitor; or
modulators of nuclear receptors for example LXR, FXR, RXR, and RORalpha;
an SSRI;
a serotonin antagonist;
or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

Therefore in an additional feature of the invention, there is provided a method for for the treatment of obesity and its associated complications in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section, or a pharmaceutically acceptable salt, solvate, solvate of such a salt or a prodrug thereof.

Therefore in an additional feature of the invention, there is provided a method of treating hyperlipidemic conditions in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt thereof, and a compound from one of the other classes of compounds described in this combination section or a pharmaceutically acceptable salt, solvate, solvate of such a salt or a prodrug thereof, in association with a pharmaceutically acceptable diluent or carrier.

According to a further aspect of the present invention there is provided a kit comprising a compound of formula I, or a pharmaceutically acceptable salt thereof, and a compound from one of the other classes of compounds described in this combination section or a pharmaceutically acceptable salt, solvate, solvate of such a salt or a prodrug thereof.

According to a further aspect of the present invention there is provided a kit comprising:
a) a compound of formula I, or a pharmaceutically acceptable salt thereof, in a first unit dosage form;
b) a compound from one of the other classes of compounds described in this combination section or a pharmaceutically acceptable salt, solvate, solvate of such a salt or a prodrug thereof; in a second unit dosage form; and
c) container means for containing said first and second dosage forms.

According to a further aspect of the present invention there is provided a kit comprising:
a) a compound of formula I, or a pharmaceutically acceptable salt thereof, together with a pharmaceutically acceptable diluent or carrier, in a first unit dosage form;
b) a compound from one of the other classes of compounds described in this combination section or a pharmaceutically acceptable salt, solvate, solvate of such a salt or a prodrug thereof, in a second unit dosage form; and
c) container means for containing said first and second dosage forms.

According to another feature of the invention there is provided the use of a compound of the formula I, or a pharmaceutically acceptable salt thereof, and one of the other compounds described in this combination section, or a pharmaceutically acceptable salt, solvate, solvate of such a salt or a prodrug thereof, in the manufacture of a medicament for use in the the treatment of obesity and its associated complications in a warm-blooded animal, such as man.

According to another feature of the invention there is provided the use of a compound of the formula I, or a pharmaceutically acceptable salt thereof, and one of the other compounds described in this combination section, or a pharmaceutically acceptable salt, solvate, solvate of such a salt or a prodrug thereof, in the manufacture of a medicament for use in the treatment ofhyperlipidaemic conditions in a warm-blooded animal, such as man.

According to a further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration of an effective amount of one of the other compounds described in this combination section, or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

Furthermore, a compound of the invention may also be combined with therapeutic agents that are useful in the treatment of disorders or conditions associated with obesity (such as type II diabetes, metabolic syndrome, dyslipidemia, impaired glucose tolerance, hypertension, coronary heart disease, non-alcoholic steatorheic hepatitis, osteoarthritis and some cancers) and psychiatric and neurological conditions.

### Pharmacological Activity

Compounds of the present invention are active against the receptor product of the CB1 gene. The affinity of the compounds of the invention for central cannabinoid receptors is demonstrable in methods described in Devane et al , Molecular Pharmacology, 1988, 34,605 or those described in WO01/70700 or EP 656354. Alternatively the assay may be performed as follows.
10µg of membranes prepared from cells stably transfected with the CB1 gene were suspended in 200µl of 100mM NaCl, 5mM MgCl₂, 1mM EDTA, 50mM HEPES (pH 7.4), 1mM DTT, 0.1% BSA and 100µM GDP. To this was added an EC80 concentration of agonist (CP55940), the required concentration of test compound and 0.1µCi [³⁵S]-GTPγS.
The reaction was allowed to proceed at 30°C for 45 min. Samples were then transferred on to GF/B filters using a cell harvester and washed with wash buffer (50mM Tris (pH 7.4), 5mM MgCl₂, 50mM NaCl). Filters were then covered with scintilant and counted for the amount of [³⁵S]-GTPγS retained by the filter.
Activity is measured in the absence of all ligands (minimum activity) or in the presence of an EC80 concentration of CP55940 (maximum activity). These activities are set as 0% and 100% activity respectively. At various concentrations of novel ligand, activity is calculated as a percentage of the maximum activity and plotted. The data are fitted using the equation y=A+((B-A)/1+((C/x) ÙD)) and the IC50 value determined as the concentration required to give half maximal inhibition of GTPγS binding under the conditions used.

The compounds of the present invention are active at the CB1 receptor (IC50 <1 micromolar). Most preferred compounds have IC50 <200 nanomolar.
The compounds of the invention are believed to be selective CB1 antagonists.

### Examples

### Abbreviations

DCM - dichloromethane
DMF - dimethylformamide
DMAP - 4-dimethylaminopyridine
EDC - 1-(3-dimethylaminopropyl)-3-ethylcarbodiimide
TEA - triethylamine
TFA - trifluoroacetic acid
DMSO-dimethyl sulfoxide
DEA - Diethylamine
PCC - Pyridinium chlorochromate
PyBOP - benzotriazol-1-yl-oxytri-pyrrolidinophosphonium hexafluorophosphate
HBTU - *O*-Benzotriazol-1-yl-*N,N,N',N'*-tetramethyluronium Hexafluorophosphate
DAST-(diethyl amino)sulphur trifluoride
DIEA - *N,N*-diisopropylethylamine
   - t: triplet
   - s: singlet
   - d: doublet
   - q: quartet
   - qvint: quintet
   - m: multiplet
   - br: broad
   - bs: broad singlet
   - dm: doublet of multiplet
   - bt: broad triplet
   - dd: doublet of doublet

### General Experimental Procedures

Mass spectra were recorded on either a Micromass ZQ single quadrupole or a Micromass LCZ single quadrupole mass spectrometer both equipped with a pneumatically assisted electrospray interface (LC-MS). ¹H NMR measurements were performed on either a Varian Mercury 300 or a Varian Inova 500, operating at ¹H frequencies of 300 and 500 MHz respectively. Chemical shifts are given in ppm with CDCl₃ as internal standard. CDCl₃ is used as the solvent for NMR unless otherwise stated. Purification was performed on a semipreparative HPLC with a mass triggered fraction collector, Shimadzu QP 8000 single quadrupole mass spectrometer equipped with 19 x 100 mm C8 column. The mobile phase used was, if nothing else is stated, acetonitrile and buffer (0.1 M NH₄Ac:acetonitrile 95:5).

For isolation of isomers, a Kromasil CN E9344 (250 x 20 mm i.d.) column was used. Heptane:ethyl acetate:DEA 95:5:0.1 was used as mobile phase (1 ml/min). Fraction collection was guided using a UV-detector (330 nm).

### Examples of the Invention

### Example 1

### Step A 5,6-bis(4-chlorophenyl)pyrazine-2,3-dicarbonitrile

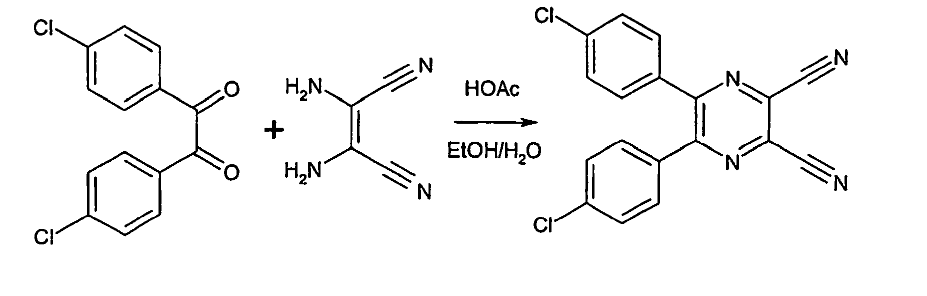

A mixture of 1,2-bis(4-chlorophenyl)ethane-1,2-dione, (20 g, 71.65 mmol), diaminomaleonitrile (8.5 g, 78.82 mmol) and acetic acid (6 ml) in ethanol (140 ml) and water (93 ml) was heated at 75 °C overnight. The reaction mixture was cooled, and water was added. The precipitate was filtered and washed with ethanol and then ether. The crude product was dissolved in DCM and treated with activated charcoal, then filtered through celite. The solid was recrystallized from DCM/ethanol to give the title compound (17.3 g, 69%) as a solid.
¹H NMR (400 MHz) δ 7.49 (d, 4H), 7.38 (d, 4H).

### Step B 5,6-bis(4-chlorophenyl)pyrazine-2,3-dicarboxylic acid

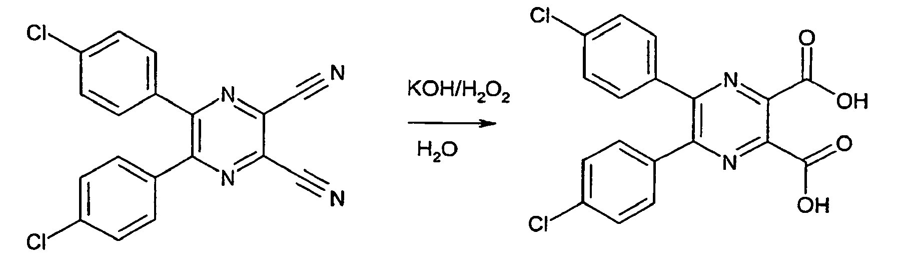

To a suspension of 5,6-bis(4-chlorophenyl)pyrazine-2,3-dicarbonitrile, Example 1, Step A (16.3 g, 46.28 mmol) and KOH (26 g, 463 mmol) in water (84 ml) was added hydrogen peroxide (35%, 19 ml) followed by a few drops of nonanol to reduce foaming. The reaction mixture was boiled under reflux for 2h, cooled and washed with ether and acidified to pH 4 with 2M HCl. The precipitate was filtered, washed with water and dried under reduced pressure to give the crude product which was esterified by refluxing in hydrogen chloride/methanol (100 ml) followed by HPLC purification, giving 12.85 g of the methyl ester. The resulting methyl ester in acetonitrile (140 ml) and water (90 ml) was treated with lithium hydroxide (2.95 g, 0.123 mmol) at ambient temperature for 1.5 h. The acetonitrile was removed under reduced pressure and the aqueous solution was washed with diethyl ether. Acidification with hydrochloric acid (2M) gave the title compound (11.8 g, 66% mmol) as a pale yellow solid which was isolated by filtration. ¹H NMR (400 MHz) δ 7.51 (d, 4H), 7.41 (d, 4H). MS *m*/*z* 389, 391 (M+H)⁺.

### Step C 2,3-bis(4-chlorophenyl)furo[3,4-b]pyrazine-5,7-dione

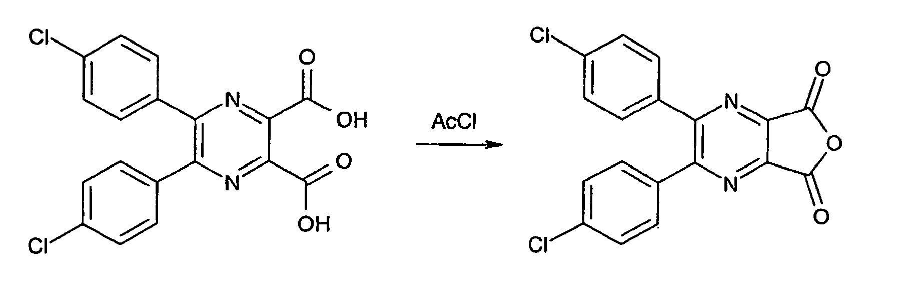

A mixture of 5,6-bis(4-chlorophenyl)pyrazine-2,3-dicarboxylic acid, Example 1, Step B (6.7 g, 17.30 mmol) and acetyl chloride (20 ml) was boiled under reflux overnight. The excess of acetyl chloride was removed under reduced pressure to give the title compound (6.2 g, 97%) as a solid.
¹H NMR (400 MHz) δ 7.51 (d, 4H), 7.41 (d, 4H).

### Step D 5,6-bis(-4-chlorophenyl)-3-{[(4-fluorobenzyl)amino]carbonyl}pyrazine-2-carboxylic acid:

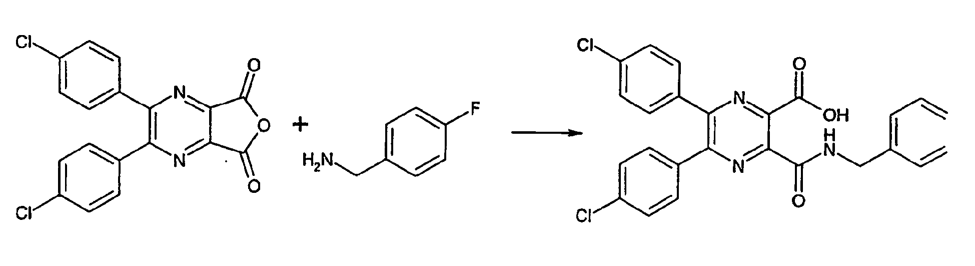

(4-Fluorobenzyl)amine (202 mg, 1.61 mmol) was mixed with 2,3-bis(4-chlorophenyl)furo[3,4-*b*]pyrazine-5,7-dione Ex. 1, Step C (544 mg, 1.47 mmol) in acetonitrile (10 ml). The reaction mixture was left at room temperature for 60 h. Evaporation followed by purification by HPLC gave the pure compound (700 mg, 96%). ¹H NMR (500 MHz, CD₃OD) δ 7.55-7.46 (m, 4H), 7.46-7.39 (m, 2H), 7.39-7.34 (m, 4H), 7.08-7.01 (m, 2H) 4.60 (s, 2H).
MS *m*/*z* calcd for [C₂₅H₁₇Cl₂N₃O₃F]H⁺ 496.0631, found 496.0643 (M+H)⁺

### Step E 2,3-bis(4-chlorophenyl)-6-(4-fluorobenzyl)-5H-pyrrolo[3,4-b]pyrazine-5,7(6H)-dione :

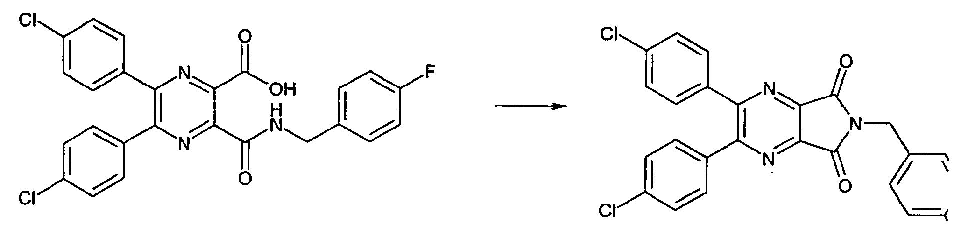

5,6-Bis(4-chlorophenyl)-3-{[(4-fluorobenzyl)amino]carbonyl}pyrazine-2-carboxylic acid, Ex.1, Step D (220 mg, 0.443 mmol) was dissolved in methylene chloride (5 ml). DMF (20 microlitres) was added and then oxalyl chloride (1 ml). After 1 hour at room temperature the solvent was removed in vacuo and the residue was purified by preparative HPLC to give the title compound (156 mg, 74%).
¹H NMR (500 MHz, CDCl₃) δ 7.52-7.42 (m, 6H), 7.36-7.30 (d, 4H), 7.05-6.96 ("t", 2H), 4.95 (s, 2H).
MS *m*/*z* calcd for [C₂₅H₁₄Cl₂FN₃O₂]H⁺ 478.0528, found 478.0559 (M+H)⁺.

### Example 2

### 2 3-Bis(4-chlorophenyl)-6-(pyridin-4-ylmethyl)-5H-pyrrolol[3,4-b]pyrazine-5,7(6H)-dione

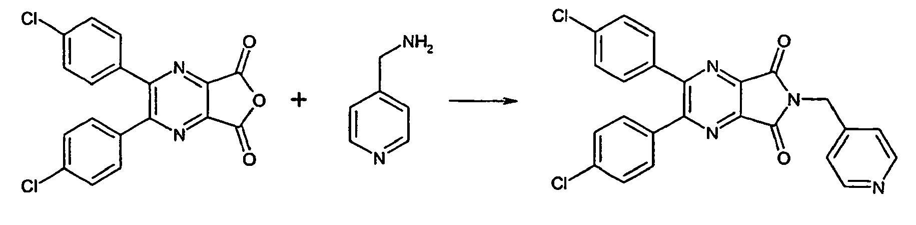

2,3-Bis(4-chlorophenyl)furo[3,4-*b*]pyrazine-5,7-dione, EX1, Step C (214 mg, 0.58 mmol) was dissolved in methylene chloride (3 ml) followed by (pyridin-4-ylmethyl)amine (62 mg, 0.58 mmol). After 5 days at room temperature, DMF (20 microlitres) and thionyl chloride (1 ml) were added. The solvent was removed in vacuo after 1 hour and the residue was purified by preparative HPLC to give the title compound (104 mg, 39%). ¹H NMR (400 MHz, CDCl₃) δ 8.56 (broad s, 2H), 7.45 (d, 4H), 7.35-7.29 (m, 6H), 4.97 (s, 2H).
MS *m*/*z* calcd for [C₂₄H₁₄Cl₂N₄O₂]H⁺ 461.0572, found 461.0585 (M+H)⁺

### Example 3

### StepA 5,6-bis(4-chlorophenyl)-3-[(piperidin-1-ylamino)carbonyl]pyrazine-2-carboxylic acid:

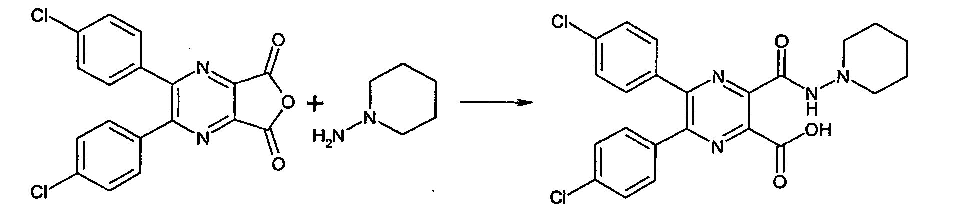

A solution of piperidin-1-amine (292 mg, 2.91 mmol) in acetonitrile (10 ml) was added to a solution of 2,3-bis(4-chlorophenyl)furo[3,4-*b*]pyrazine-5,7-dione, Ex1, Step C (1.03 g, 2.78 mmol) in acetonitrile (10 ml). After 2 hours the solvent was removed in vacuo and the title compound could be isolated by crystallization from acetonitrile. The yield was 807 mg (62%).
¹H NMR (400 MHz) δ 7.55 (d, 2H), 7.46 (d, 2H), 7.37 (d, 2H), 7.31 (d, 2H), 3.05-2.97 (m, 4H), 1.84-1.77 (m, 4H), 1.54-1.46 (m, 2H).
MS *m*/*z* calcd for [C₂₃H₂₁Cl₂N₄O₃]H⁺ 471.0991, found 471.0994 (M+H)⁺.

### Step B2,3-bis(4-chlorophenyl)-6-piperidin-1-yl-5H-pyrrolo[3,4-b]pyrazine-5,7(6H)-dione:

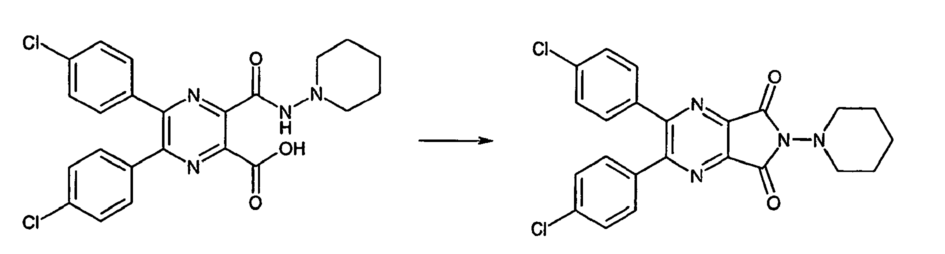

Oxalyl chloride (0.4 ml) was added to 5,6-bis(4-chlorophenyl)-3-[(piperidin-1-ylamino)carbonyl]pyrazine-2-carboxylic acid (229 mg, 0.486 mmol) in methylene chloride (10 ml). After 10 minutes water was added and then sodium carbonate solution.

The organic phase was washed with water and the solvent was evaporated. The product was isolated by crystallisation from methylene chloride/hexane (92 mg, 42%).
¹H NMR (400 MHz) δ 7.47 (d, 4H), 7.34 (d, 4H), 3.41-3.36 (m, 4H), 1.84-1.76 (m, 4H), 1.60-1.50 (m, 2H).
MS *m*/*z* calcd for [C₂₃H₁₈Cl₂N₄O₂]H⁺ 453.0918, found 453.0885 (M+H)⁺.

### Example 4

### STEP A 3-[(tert-butylamino)carbonyl]-5,6-bis(4-chlorophenyl)pyrazine-2-carboxylic acid

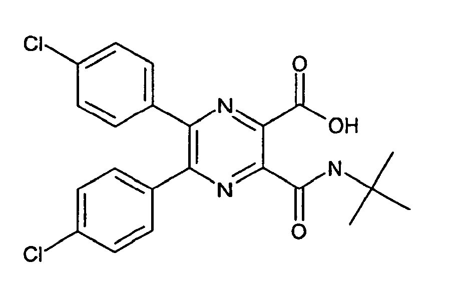

To a solution of 2,3-bis(4-chlorophenyl)furo[3,4-*b*]pyrazine-5,7-dione, Ex.1, stepC (500 mg, 1.35 mmol) in acetonitrile (10 ml) was added *tert*-butylamine (99 mg, 1.35 mmol). The reaction mixture was stirred in room temperature for 1h 15min to give the subtitle compound. ¹H NMR (400 MHz) δ 7.46-7.41 (m, 4H), 7.38-7.34 (m, 4H), 1.41 (s, 9H).
MS *m*/*z* 444 (M+H)⁺.

### Step B 6-tert-butyl-2,3-bis(4-chlorophenyl)-5H-pyrrolo[3,4-b]pyrazine-5,7(6H)-dione

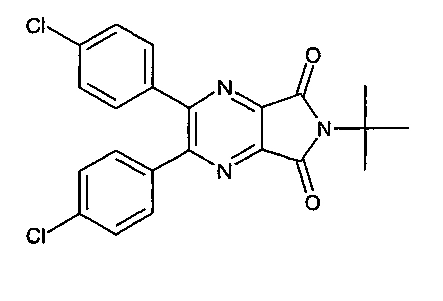

To a solution of 3-[(*tert*-butylamino)carbonyl]-5,6-bis(4-chlorophenyl)pyrazine-2-carboxylic, Ex. 4, Step A (100 mg, 0.23mmol) in methylene chloride (5 ml) were added oxalyl chloride (2.5 ml) and a few drops of *N,N*-dimethylformamide. The reaction mixture was stirred in room temperature for 2hours. The mixture was then filtered through silica and preparatory HPLC gave the title compound as a solid. ¹H NMR (400 MHz) δ 7.45 (d, 4H), 7.32 (d, 4H), 1.75 (s, 9H).
MS *m*/*z* 426 (M+H)⁺.

## Claims

1. A compound of formula (I) 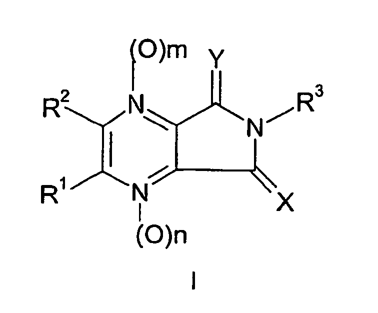 and pharmaceutically acceptable salts thereof, in which
R¹ and R² independently represent phenyl, thienyl, pyridyl, C₁₋₁₀alkyl, C₁₋₁₀alkoxy or C₃₋₁₅cycloalkyl ;
R³ represents a C₁₋₁₅alkyl group, C₃₋₁₅cycloalkyl, a phenylC₁₋₄alkyl group, a heteroarylgroup, a heteroarylC₁₋₄alkyl group, or a group R⁴(CH₂)ₙ- in which R⁴ represents a saturated or partially unsaturated 5 to 8 membered heterocyclic group containing one or more heteroatoms selected from nitrogen, oxygen or sulphur and n is 0, 1, 2, 3 or 4;
X and Y independently represent O or S;
m and n independently represent 0 or 1;
wherein each of R¹, R², R³ and R⁴ is optionally substituted by one, two or three groups represented by Z wherein Z represents a C₁₋₆alkyl group optionally substituted by one or more fluoro, a C₁₋₆alkoxy group optionally substituted by one or more fluoro, hydroxy, halo, trifluoromethylsulphonyl, benzyl, nitro, amino, mono or di C₁₋₄alkylamino, mono or di C₁₋₃alkylamido, C₁₋₃alkylsulphonyl, C₁₋₆alkoxycarbonyl, carboxy, cyano, carbamoyl, mono or di C₁₋₃alkyl carbamoyl, sulphamoyl or acetyl.

2. A compound of formula (IA) 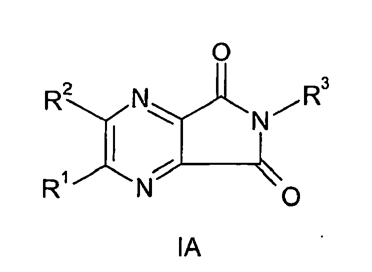 and pharmaceutically acceptable salts thereof, in which
R¹ and R² independently represent phenyl, thienyl, pyridyl, C₁₋₁₀alkyl, C₁₋₁₀alkoxy or C₃₋₁₅cycloalkyl ;
R³ represents a C₁₋₁₅alkyl group, C₃₋₁₅cycloalkyl, a phenylC₁₋₄alkyl group, a heteroaryl group, a heteroarylC₁₋₄alkyl group, or a group R⁴(CH₂)ₙ- in which R⁴ represents a saturated or partially unsaturated 5 to 8 membered heterocyclic group containing one or more heteroatoms selected from nitrogen, oxygen or sulphur and n is 0, 1, 2, 3 or 4;
X and Y independently represent O or S;
m and n independently represent 0 or 1;
wherein each of R¹, R², R³ and R⁴ is optionally substituted by one, two or three groups represented by Z wherein Z represents a C₁₋₆alkyl group optionally substituted by one or more fluoro, a C₁₋₆alkoxy group optionally substituted by one or more fluoro, hydroxy, halo, trifluoromethylsulphonyl, benzyl, nitro, amino, mono or di C₁₋₄alkylamino, mono or di C₁₋₃alkylamido, C₁₋₃alkylsulphonyl, C₁₋₆alkoxycarbonyl, carboxy, cyano, carbamoyl, mono or di C₁₋₃alkyl carbamoyl, sulphamoyl or acetyl.

3. A compound selected from one or more of the following:
2,3-bis(4-chlorophenyl)-6-(4-fluorobenzyl)-5H-pyrrolo[3,4-*b*]pyrazine-5,7(6*H*)-dione;
2,3-bis(4-chlorophenyl)-6-(pyridin-4-ylmethyl)-5*H*-pyrrolo[3,4-*b*]pyrazine-5,7(6*H*)-dione;
2,3-bis(4-chlorophenyl)-6-piperidin-1-yl-5*H*-pyrrolo[3,4-*b*]pyrazine-5,7(6*H*)-dione; or
6-*tert*-butyl-2,3-bis(4-chlorophenyl)-5*H*-pyrrolo[3,4-*b*]pyrazine-5,7(6*H*)-dione
as well as pharmaceutically acceptable salts thereof.

4. A compound of formula I as claimed in any one of claims 1 to 3 for use as a medicament.

5. A pharmaceutical formulation comprising a compound of formula I as claimed in any one of claims 1 to 3 and a pharmaceutically acceptable adjuvant, diluent or carrier.

6. Use of a compound of formula I as claimed in any one of claims 1 to 3 in the preparation of a medicament for the treatment or prophylaxis of obesity, psychiatric disorders such as psychotic disorders, schizophrenia and bipolar disorders, anxiety, anxio-depressive disorders, depression, cognitive disorders, memory disorders, obsessive-compulsive disorders, anorexia, bulimia, attention disorders, epilepsy, and related conditions, and neurological disorders such as dementia, Parkinson's Disease, Huntington's Chorea and Alzheimer's Disease, immune, cardiovascular, reproductive and endocrine disorders, septic shock, diseases related to the respiratory and gastrointestinal systems, and extended abuse, addiction and/or relapse indications.

7. A compound as defined in any one of claims 1 to 3 for use in the treatment of obesity.

## Patentansprüche

1. Verbindungen der Formel (I) 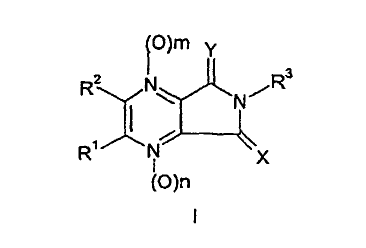 und deren pharmazeutisch annehmbare Salze, in denen
R¹ und R² unabhängig voneinander für Phenyl, Thienyl, Pyridyl, C₁₋₁₀-Alkyl, C₁₋₁₀-Alkoxy oder C₃₋₁₅-Cycloalkyl stehen;
R³ für eine C₁₋₁₅-alkylgruppe, C₃₋₁₅-Cycloalkyl, eine Phenyl-C₁₋₄-Alkylgruppe, eine Heteroarylgruppe, eine Heteroaryl-C₁₋₄-alkylgruppe oder eine Gruppe
R⁴(CH₂)ₙ steht, wobei R⁴ für eine gesättigte oder teilweise ungesättigte 5- bis 8-gliedrige heterocyclische Gruppe mit einem oder mehreren Heteroatomen ausgewählt aus Stickstoff, Sauerstoff und Schwefel steht und n für 0, 1, 2, 3 oder 4 steht;
X und Y unabhängig voneinander für O oder S stehen;
m und n unabhängig voneinander für 0 oder 1 stehen ;
wobei R¹, R², R³ und R⁴ jeweils gegebenenfalls durch eine, zwei oder drei durch Z wiedergegebene Gruppen substituiert sind, wobei Z für eine C₁₋₆-Alkylgruppe, gegebenenfalls substituiert durch ein oder mehrere Fluor, eine C₁₋₆-Alkoxygruppe, gegebenenfalls substituiert durch ein oder mehrere Fluor, Hydroxy, Halogen, Trifluormethylsulfonyl, Benzyl, Nitro, Amino, Mono- oder Di-C₁₋₄-alkylamino, Mono- oder Di-C₁₋₃-alkylamido, C₁₋₃-Alkylsulfonyl, C₁₋₆-Alkoxycarbonyl, Carboxyl, Cyano, Carbamoyl, Mono- oder Di-C₁₋₃-alkylcarbamoyl, Sulfamoyl oder Acetyl, steht.

2. Verbindungen der Formel (IA) 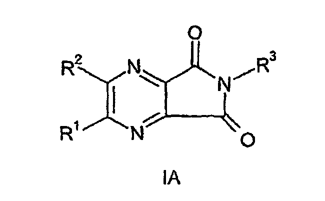 und und deren pharmazeutisch annehmbare Salze, in denen
R¹ und R² unabhängig voneinander für Phenyl, Thienyl, Pyridyl, C₁₋₁₀-Alkyl, C₁₋₁₀-Alkoxy oder C₃₋₁₅-Cycloalkyl stehen;
R³ für eine C₁₋₁₅-alkylgruppe, C₃₋₁₅-Cycloalkyl, eine Phenyl-C₁₋₄-Alkylgruppe, eine Heteroarylgruppe, eine Heteroaryl-C₁₋₄-alkylgruppe oder eine Gruppe R⁴(CH₂)ₙ steht, wobei R⁴ für eine gesättigte oder teilweise ungesättigte 5- bis 8-gliedrige heterocyclische Gruppe mit einem oder mehreren Heteroatomen ausgewählt aus Stickstoff, Sauerstoff und Schwefel steht und n für 0, 1, 2, 3 oder 4 steht;
X und Y unabhängig voneinander für O oder S stehen;
m und n unabhängig voneinander für 0 oder 1 stehen ;
wobei R¹, R², R³ und R⁴ jeweils gegebenenfalls durch eine, zwei oder drei durch Z wiedergegebene Gruppen substituiert sind, wobei Z für eine C₁₋₆-Alkylgruppe, gegebenenfalls substituiert durch ein oder mehrere Fluor, eine C₁₋₆-Alkoxygruppe, gegebenenfalls substituiert durch ein oder mehrere Fluor, Hydroxy, Halogen, Trifluormethylsulfonyl, Benzyl, Nitro, Amino, Mono- oder Di-C₁₋₄-alkylamino, Mono- oder Di-C₁₋₃-alkylamido, C₁₋₃-Alkylsulfonyl, C₁₋₆-Alkoxycarbonyl, Carboxyl, Cyano, Carbamoyl, Mono- oder Di-C₁₋₃-alkylcarbamoyl, Sulfamoyl oder Acetyl steht.

3. Verbindungen ausgewählt aus einer oder mehreren der folgenden:
2,3-Bis(4-chlorphenyl)-6-(4-fluorbenzyl)-5H-pyrrol[3,4-b]pyrazin-5,7(6H)-dion;
2,3-Bis(4-chlorphenyl)-6-(pyridin-4-ylmethyl)-5H-pyrrol[3,4-b]pyrazin-5,7(6H)-dion;
2,3-Bis(4-chlorphenyl)-6-piperidin-1-yl-5H-pyrrol[3,4-b]pyrazin-5,7(6H)-dion; oder
6-tert.-Butyl-2,3-bis(4-chlorphenyl)-5H-pyrrol[3,4-b]pyrazin-5,7(6H)-dion
sowie deren pharmazeutisch annehmbare Salze.

4. Verbindungen der Formel I nach einem der Ansprüche 1 bis 3 zur Verwendung als Medikament.

5. Pharmazeutische Formulierung, enthaltend eine Verbindung der Formel I nach einem der Ansprüche 1 bis 3 und ein pharmazeutisch annehmbares Adjuvans, ein pharmazeutisch annehmbares Verdünnungsmittel oder einen pharmazeutisch annehmbaren Träger.

6. Verwendung einer Verbindung der Formel I nach einem der Ansprüche 1 bis 3 bei der Herstellung eines Medikaments zur Behandlung oder Prophylaxe von Obesitas, psychiatrischen Erkrankungen, wie psychotischen Erkrankungen, Schizophrenie und manisch-depressiver Psychose, Angstzuständen, anxiodepressiven Erkrankungen, Depression, kognitiven Störungen, Gedächtnisstörungen, Zwangsneurosen, Anorexie, Bulimie, Aufmerksamkeitsstörungen, Epilepsie und verwandten Leiden, und neurologischen Erkrankungen wie Demenz, Parkinson-Krankheit, Chorea Huntington und Alzheimer-Krankheit, Erkrankungen des Immunsystems, des Herzkreislaufsystems, der Fortpflanzungsorgane und des Endokrinsystems, septischem Schock, mit den Atemwegen und dem Magen-Darm-Trakt in Zusammenhang stehenden Erkrankungen und Indikationen von anhaltendem Substanzmißbrauch, Sucht und/oder Rückfall.

7. Verbindungen nach einem der Ansprüche 1 bis 3 zur Verwendung bei der Behandlung von Obesitas.

## Revendications

1. Composé de la formule 1 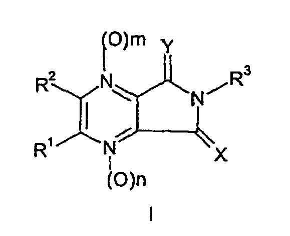 et des sels pharmaceutiquement acceptables de celui-ci, dans laquelle
R¹ et R² représentent indépendamment un groupe phényle, un groupe thiényle, un groupe pyridyle, un groupe alkyle en C₁ à C₁₀, un groupe alcoxy en C₁ à C₁₀ ou un groupe cycloalkyle en C₃ à C₁₅ ;
R3 représente un groupe alkyle en C₁ à C₁₅, un groupe cycloalkyle en C₃ à C₁₅, un groupe phényle-alkyle en C₁ à C₄, un groupe hétéroaryle, un groupe hétéroaryle-alkyle en C₁ à C₄ ou un groupe R⁴(CH₂)ₙ- dans lequel R₄ représente un groupe hétérocyclique à 5 à 8 chaînons saturé ou partiellement insaturé qui comprend un ou plusieurs hétéroatomes sélectionnés parmi l'atome d'azote, l'atome d'oxygène ou l'atome de soufre et n vaut 0, 1, 2, 3 ou 4 ;
X et Y représentent indépendamment un O ou un S ;
m et n représentent indépendamment 0 ou 1 ;
dans laquelle chacun de R¹, R², R³ et R⁴ est éventuellement substitué par un, deux ou trois groupes représentés par Z, dans lesquels Z représente un groupe alkyle en C₁ à C₆ éventuellement substitué par un ou plusieurs fluoro, alcoxy en C₁ à C₆ éventuellement substitué par un ou plusieurs fluoro, hydroxy, halo, trifluorométhylsulfonyle, benzyle, nitro, amino, mono ou dialkylamino en C₁ à C₄, mono ou dialkylamido en C₁ à C₃, alkylsulfonyle en C₁ à C₃, alcoxycarbonyle en C₁ à C₆, carboxy, cyano, carbamoyle, mono ou dialkylcarbamoyle en C₁ à C₃, sulfamoyle ou acétyle.

2. Composé de la formule (IA) 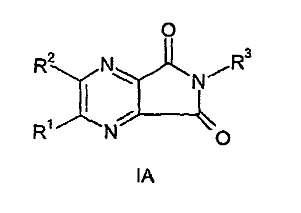 et des sels pharmaceutiquement acceptables de celui-ci, dans laquelle
R¹ et R² représentent indépendamment un groupe phényle, un groupe thiényle, un groupe pyridyle, un groupe alkyle en C₁ à C₁₀, un groupe alcoxy en C₁ à C₁₀ ou un groupe cycloalkyle en C₃ à C₁₅ ;
R3 représente un groupe alkyle en C₁ à C₁₅, un groupe cycloalkyle en C₃ à C₁₅, un groupe phényle-alkyle en C₁ à C₄, un groupe hétéroaryle, un groupe hétéroaryle-alkyle en C₁ à C₄ ou un groupe R⁴(CH₂)ₙ- dans lequel R₄ représente un groupe hétérocyclique à 5 à 8 chaînons saturé ou partiellement insaturé qui comprend un ou plusieurs hétéroatomes sélectionnés parmi l'atome d'azote, l'atome d'oxygène ou l'atome de soufre et n vaut 0, 1, 2, 3 ou 4 ;
X et Y représentent indépendamment un O ou un S ;
m et n représentent indépendamment 0 ou 1 ;
dans laquelle chacun de R¹, R², R³ et R⁴ est éventuellement substitué par un, deux ou trois groupes représentés par Z, dans lesquels Z représente un groupe alkyle en C₁ à C₆ éventuellement substitué par un ou plusieurs fluoro, alcoxy en C₁ à C₆, éventuellement substitué par un ou plusieurs fluoro, hydroxy, halo, trifluorométhylsulfonyle, benzyle, nitro, amino, mono ou dialkylamino en C₁ à C₄, mono ou dialkylamido en C₁ à C₃, alkylsulfonyle en C₁ à C₃, alcoxycarbonyle en C₁ à C₆, carboxy, cyano, carbamoyle, mono ou dialkylcarbamoyle en C₁ à C₃, sulfamoyle ou acétyle.

3. Composé sélectionné parmi un ou plusieurs des produits suivants :
2,3-bis(4-chlorophényl)-6-(4-fluorobenzyl)-5H-pyrrolo[3,4-b]pyrazine-5,7(6H)-dione ;
2,3-bis(4-chlorophényl)-6-(pyridine-4-ylméthyl)-5H-pyrrolo[3,4-b]pyrazine-5,7(6H)-dione ;
2,3-bis(4-chlorophényl)-6-pipéridine-1-yl-5H-pyrrolo[3,4-b]pyrazine-5,7(6H)-dione ; ou
6-tert-butyl-2,3-bis(4-chlorophényl)-5H-pyrrolo[3,4-b]pyrazine-5,7(6H)-dione,
ainsi que des sels pharmaceutiquement acceptables de ceux-ci.

4. Composé de la formule I selon l'une quelconque des revendications 1 à 3 pour utilisation comme un médicament.

5. Formulation pharmaceutique comprenant un composé de la formule I selon l'une quelconque des revendications 1 à 3 et un adjuvant, un diluant ou un vecteur pharmaceutiquement acceptable.

6. Utilisation d'un composé de la formule I selon l'une quelconque des revendications 1 à 3 dans la préparation d'un médicament pour le traitement ou la prophylaxie de l'obésité, de troubles mentaux tels que des troubles psychotiques, des troubles schizophréniques et bipolaires, l'anxiété, les troubles anxio-dépressifs, la dépression, les troubles cognitifs, les troubles de la mémoire, les troubles obsessionnels-compulsifs, l'anorexie, la boulimie, les troubles d'attention, l'épilepsie et les troubles apparentés, et les troubles neurologiques tels que la démence, la maladie de Parkinson, la chorée de Huntington et la maladie d'Alzheimer, les troubles immunitaires, cardiovasculaires, reproductifs et endocriniens, le choc septique, les maladies liées aux systèmes respiratoires et gastro-intestinaux et les indications d'abus excessifs, de toxicomanie ou de récidives.

7. Composé tel que défini dans l'une quelconque des revendications 1 à 3 pour utilisation dans le traitement de l'obésité.
